# EUROPEAN PATENT APPLICATION

(11) **EP 1 932 538 A1**
(43) Date of publication of application: **18.06.2008**
(21) Application number: 06783149.5
(22) Date of filing: 25.08.2006
(51) Int. Cl.: A61K 39/00, A61K 47/34, A61K 47/36, A61K 47/42, A61K 47/48, A61P 37/08

(54) **BIODEGRADABLE NANOPARTICLE HAVING T-CELL RECOGNIZABLE EPITOPE PEPTIDE IMMOBILIZED THEREON OR ENCAPSULATED THEREIN**

(30) Priority: 25.08.2005 JP 2005243997
(71) Applicant: TAIHO PHARMACEUTICAL COMPANY LIMITED, Chiyoda-ku, Tokyo 101-8444 (JP); Akashi, Mitsuru, Suita-shi, Osaka 565-0824 (JP)
(72) Inventor: AKASHI, Mitsuru, Suita-shi, Osaka 565-0824 (JP); IKIZAWA, Koichi, Hanno-shi, Saitama 357-8527 (JP)
(74) Representative: Winter, Brandl, Fürniss, Hübner Röss, Kaiser, Polte Partnerschaft Patent- und Rechtsanwaltskanzlei
(86) International application number: PCT/JP2006/317288
(87) International publication number: WO 2007/024026

(57) **Abstract**

A biodegradable nanoparticle having a T cell recognizable epitope peptide immobilized thereon or encapsulated therein of the present invention is usable as a safe and effective immunotherapeutic agent, and is useful as an immunotherapeutic agent for treating, for example, pollinosis, year-round nasal allergic disease and seasonal nasal allergic disease.

## Description

### TECHNICAL FIELD

The present invention relates to biodegradable nanoparticles having immobilized thereon or encapsulated therein a T cell recognizable epitope peptide, more particularly a T cell recognizable epitope peptide of pollinosis patients, and/or to an immunotherapeutic agent comprising the nanoparticle.

### BACKGROUND ART

The immunotherapy for pollinosis is attributable to the finding in 1910's that injections of an extract of pollen to pollinosis patients in amounts gradually increasing from a small amount were effective. This method, which is termed also the desensitization therapy, has since been found empirically effective and practiced widely. While this immunotherapy has been accepted as the sole therapy of which a complete cure can be expected unlike drug therapies, the treatment is likely to produce side effects such as anaphylaxis since the antigen used is a pollen extract. Accordingly, the therapy has the problem that the extract can be administered only in very small amounts to suppress the development of the side effect, and the period of administration is as long as several years, therefore has found limited clinical use.

It is known that the production of cytokines such as interleukin-4, -5 or -13 or like Th2 cytokine is greater in patients with allergic diseases such as asthma and pollinosis than in healthy persons, and is related closely to the onset or development of the symptoms. On the other hand, it is also known that in patients treated by desensitization therapy, the cytokine production pattern of peripheral blood lymphocytes changes from Th2 cytokine-predominance to the predominance of Th1 cytokine typical of which is IFN-γ almost without diminishing immune response. The mechanisms of improving the symptom by immunotherapy appear to be suppression of the production of Th2 cytokine and increased production of Th1 cytokine (Nonpatent Literature 1, 2, 3).

Cryj1 and Cryj2 are known as main allergens of cedar pollen (Nonpatent Literature 4, 5, 6). It has been reported that at least 90% of patients with cedar pollinosis have specific IgE antibody to each of Cryj1 and Cryj2 (Nonpatent Literature 7).

Generally, allergens are captured by antigen-presenting cells such as macrophages and dendritic cells, thereafter digested, and the resultant fragments bind to MHC classII protein on the surface layer of the antigen-presenting cells for antigen presentation. The antigen-presenting fragments are limited to some specific regions of allergens owing to the affinity for MHC classII protein. Among these specific regions, the region to be specifically recognized by T cells is usually called a "T cell epitope", and the region to be specifically recognized by B cells is usually termed an "B cell epitope." Presently, attention has been directed toward an immunotherapy wherein peptides comprising a T cell epitope are administered. The therapy has the following advantages.
(1) The peptide, which is deficient in B cell epitope, does not react with IgE antibody which is specific to the allergen, so that side effect including anaphylaxis which is frequently experienced with conventional crude or purified allergens is unlikely to occur
(2) The period following the start of the therapy at a small dose until the dose of the peptide reaches an effective level can be much shorter than is the case with the conventional desensitization therapy (Nonpatent Literature 8).

The details of the mechanism of the peptide immunotherapy still remain to be clarified, whereas the mechanism appears to involve the following possibilities (Nonpatent Literature 3).
(1) The peptide binds directly to the antigen binding site of T cell receptor appearing on the surface of T cells to avert the stimulation of antigen and co-stimulation via the ordinary T cells-antigen presenting cells signal, leading to the possibility of inducing the inactivation (anergy) or immunological tolerance of T cells.
(2) The possibility of inducing promoted production of IgG antibody or inactivation of T cells by the administration of large quantities of the peptide.

Already reported are many T-cell recognizable epitope peptides of cedar pollinosis patients. Reportedly, animal experiments have shown that T cell recognizable epitope peptides for Cryj1 and Cryj2 which are main allergens of cedar pollen in mice, when administered before immunization with the main allergens, induce inactivation (anergy) or immunological tolerance of T cells (Nonpatent Literature 9, 10, 11, 12).

Thus, already known is the fact that T cell recognizable epitope peptides are useful as immunotherapeutic agents for cedar pollinosis. The administration of peptides to the living body generally appears to involve the following problems.
(1) The peptide is decomposed by an enzyme and therefore can not be maintained at an effective concentration (problem of stability).
(2) The digestive tract membrane is very low in permeability to the peptide, which therefore can not be administered orally without problems.
(3) If attempted for the purpose of inducing a reaction through mucosal immunity, the submucous administration of the peptide involves problems with respect to tissue retentivity.

Not only these problems can be overcome by using nanoparticles which are generally used as drug delivery systems, but it is also possible to expect a different synergic effect when T cell recognizable epitope peptide is immobilized on or encapsulated in nanoparticles, unlike the effect to be produced when the peptide is used singly as it is.

Nanoparticles can be prepared from copolymers which are different in composition or functional group and thereby given various structures. Utilizing these forms, nanoparticles have found a wide variety of applications as paint or coating materials, integration materials and medical materials such as drug delivery carriers, among which the application as medical materials has attracted special attention. For use as medical materials, nanoparticles per se and the product of the particle as decomposed or metabolized are preferably safe or nontoxic or low in toxicity. Therefore preferable are nanoparticles which are biodegradable and compatible with the living body (hereinafter referred to as "biodegradable nanoparticles").

Many examples of biodegradable nanoparticles have already been reported which include poly-D,L-lactide-co-glycolide (PLGA) (Patent Literature 1), nanoparticles made from polycyanoacrylate polymer (Patent Literature 2), nanoparticles made from poly(γ-glutamic acid) (γ-PGA), producible in large quantities using bacillus natto and having biodegradablity and living body compatibility (Patent Literature 3), and nanoparticles comprising a graft copolymer of poly(γ-glutamic acid)(γ-PGA) and phenylalanine ethyl ester (L-PAE)(Nonpatent Literature 13)

Reports have been made on animal experiments (mice) wherein PLGA was used as an application to immunotherapy with biodegradable nanoparticles having an antigen immobilized therein on or encapsulated therein (Nonpatent Literature 14, 15, 16). However, the antigens used are all main allergens (Bet vl/birch antigen, Ole el/olive antigen and phospholipase A2-coding vector/bee venom), and no reports have heretofore been made on an immunotherapeutic agent having a T cell epitope peptide immobilized thereon or encapsulated therein as in the present invention. On the other hand, a report has been made on an anti-virus therapy wherein T cell epitope peptide is encapsulated in poly(lactide-co-glycolide) (PLG) to ensure enhanced immunogenicity (Nonpatent Literature 17). The anti-virus therapy is intended to provide enhanced immunity involving increased antibody production, giving an effect exactly reverse to the foregoing immunotherapy of the invention intended to control the balance between Th1 and Th2.

As described above, no reports have been made on the use of biodegradable nanoparticles having immobilized thereon or encapsulate therein a T cell recognizable epitope peptide as an immunotherapeutic agent for allergic diseases typical of which are asthma and pollinosis.
[Nonpatent Literature 1] Clin. Exp. Allergy 25: 828-838 (1995)
[Nonpatent Literature 2] Clin. Exp. Allergy 27: 1007-1015 (1997)
[Nonpatent Literature 3] Arch. Otolaryngol. Head Neck Surg. 126: 63-70 (2000)
[Nonpatent Literature 4] J. Allergy Clin. Immunol. 71:77-86 (1983)
[Nonpatent Literature 5] FEBS Letter 239: 329-332 (1988)
[Nonpatent Literature 6] Allergy. 45: 309-312 (1990)
[Nonpatent Literature 7] Clin. Exp. Allergy 25: 848-852 (1995)
[Nonpatent Literature 8] Int. Arch. Allergy Immunol. 122: 229-237 (2000)
[Nonpatent Literature 9] Immunology 107: 517-520 (2002)
[Nonpatent Literature 10] J. Allergy Clin. Immunol. 102: 961-967 (1998)
[Nonpatent Literature 11] Eur. J. Immunol. 32: 1631-1639 (2002)
[Nonpatent Literature 12] Eur. J. Pharmacol. 510: 143-148 (2005)
[Nonpatent Literature 13] Macromolecular Bioscience 5: 598-602 (2005)
[Nonpatent Literature 14] Clin. Exp. Allergy 34: 315-321 (2004)
[Nonpatent Literature 15] J. Contrl. Release 92: 395-398 (2003)
[Nonpatent Literature 16] J. Allergy Clin. Immunol. 114: 943-950 (2004)
[Nonpatent Literature 17] J. Immunol. Methods 195: 135-138 (1996)
[Patent Literature 1] JP 1997-504027A
[Patent Literature 2] JP 1996-530157A
[Patent Literature 3] JP 2003-342367A

An object of the present invention is to provide biodegradable nanoparticles having immobilized thereon or encapsulated therein a T cell recognizable epitope peptide, more particularly a T cell recognizable epitope peptide of pollinosis patients, and/or to an immunotherapeutic agent comprising the nanoparticle.

### DISCLOSURE OF THE INVENTION

The present invention has the following features.
(1) A biodegradable nanoparticle having a T cell recognizable epitope peptide immobilized thereon or encapsulated therein.
(2) A nanoparticle according to par. (1) which comprises a nanoparticle mainly prepared from a polypeptide, polysaccharide or poly(organic acid).
(3) A nanoparticle according to par. (2) wherein the polypeptide is poly(γ-glutamic acid).
(4) A nanoparticle according to par. (2) wherein the polypeptide is a graft copolymer of poly(γ-glutamic acid) and phenylalanine ethyl ester.
(5) A nanoparticle according to any one of pars. (1) to (4) wherein the T cell recognizable epitope peptide is encapsulated in the nanoparticle.
(6) A nanoparticle according to any one of pars. (1) to (4) wherein the T cell recognizable epitope peptide is present on a surface of the nanoparticle.
(7) A nanoparticle according to any one of pars. (1) to (6) wherein the T cell recognizable epitope peptide is a cedar pollen T cell recognizable epitope peptide.
(8) An immunotherapeutic agent containing a nanoparticle according to any one of pars. (1) to (7).
(9) An immunotherapeutic agent according to par. (8) for treating and/or preventing cedar pollinosis.
(10) Use of a biodegradable nanoparticle having a T cell recognizable epitope peptide immobilized thereon or encapsulated therein for preparing an immunotherapeutic agent.
(11) Use according to par. (10) for treating and/or preventing cedar pollinosis.
(12) An immunotherapy comprising administering to a mammal an effective amount of a biodegradable nanoparticle having a T cell recognizable epitope peptide immobilized thereon or encapsulated therein.
(13) An immunotherapy according to par. (12) for treating and/or preventing cedar pollinosis.

In view of the foregoing situation, we have conducted intensive research and found that T cell epitope peptides, especially T cell recognizable epitope peptides of pollinosis patients can be immobilized on or encapsulated in biodegradable nanoparticles without degradation or decomposition so that the biodegradable nanoparticles can be used with safety efficiently as a peptide immunotherapeutic agent. Thus, the present invention has been accomplished.

The term "T cell recognizable epitope peptide" as used herein means a peptide recognizable by T cells and serving as an antigen.

By the term "immobilization" as herein used is meant direct bonding of the peptide to the nanoparticle by a covalent bond, ionic bond or intermolecular force, by adsorption or by inclusion, or bonding through a linker such as polyethylene glycol (PEG).

By the term "encapsulation" as herein used is meant direct bonding of the peptide to the interior of the nanoparticle by a covalent bond, ionic bond or intermolecular force, by adsorption or by inclusion, or bonding through a linker such as polyethylene glycol (PEG).

The present invention relates to biodegradable nanoparticles having immobilized thereon or encapsulated therein a T cell recognizable epitope peptide, more particularly a T cell recognizable epitope peptide of pollinosis patients. Various materials are usable for the biodegradable nanoparticles of the invention. These materials are known well in the art, and suitable materials can be selected for use. Since such nanoparticles are administered to the living body, it is desired that the nanoparticles themselves and the product of the particle as decomposed or metabolized be safe, nontoxic or low in toxicity. Examples of preferred materials for nanoparticles are polypeptides, polysaccharides and poly(organic acids), or mixtures of such materials. Polypeptides are more preferred.

Biodegradable nanoparticles made chiefly from a polypeptide (hereinafter referred to as "biodegradable polypeptide nanoparticles") may contain a natural amino acid, modified amino acid (e.g., esterified amino acid), synthetic amino acid, or a mixture of such acids. In view of safety and toxicity, more preferable are those comprising a natural amino acid. Examples of preferred biodegradable polypeptide nanoparticles comprising such a natural amino acid are poly(γ-glutamic acid) nanoparticles, poly(γ-lysine) nanoparticles, poly(α-L-lysine) nanoparticles, poly(α-aspartic acid) nanoparticles, etc. Further biodegradable polypeptide nanoparticles may comprise a single amino acid, or at least two amino acids. In the biodegradable polypeptide nanoparticle, all the bonds between the component amino acids may be of the same kind or different kinds. For example, all the component amino acids may be linked by peptide bond. Alternatively, the amino acids may be bonded by a linkage other than the peptide linkage locally or wholly. Amino acids may be bonded by a linker. For example, a hydrophobic amino acid may be introduced into the side chain of a hydrophilic amino acid to effect a desired hydrophilic-hydrophobic balance. Accordingly the polypeptide may be a graft copolymer of poly(γ-glutamic acid) and phenylalanine ethyl ester. The biodegradable polypeptide nanoparticle of the present invention consists mainly of a polypeptide(preferably in an amount of at least 50 wt. % when having no T cell recognizable epitope peptide immobilized thereon), the polypeptide preferably providing a skeleton. The biodegradable polypeptide nanoparticle of the invention may contain a component other than the polypeptide or amino acid, in the skeleton or other portion thereof, or need not have such component.

Biodegradable nanoparticles made chiefly from a polysaccharide (hereinafter referred to as "biodegradable polysaccharide nanoparticles") may contain a natural polysaccharide, modified polysaccharide, synthetic polysaccharide or a mixture of such polysaccharides. In view of safety and toxicity, more preferable are those comprising a natural polysaccharide. Examples of preferred biodegradable polysaccharide nanoparticles comprising such a natural polysaccharide are pullulan nanoparticles, chitosan nanoparticles, alginic acid nanoparticles, pectin nanoparticles, curdlan, nanoparticles, dextran nanoparticles, etc. Further biodegradable polysaccharide nanoparticles may comprise a single saccharide, or at least two saccharides. The biodegradable polysaccharide nanoparticle may comprise component saccharides all linked by the same bond, or the component saccharides may be linked by different bonds locally or wholly. For example, α-1,6 bonds and α-1,4 bonds may be present conjointly. The saccharides may be bonded by a linker. The biodegradable polysaccharide nanoparticle of the present invention consists mainly of a polysaccharide (preferably in an amount of at least 50 wt. % when having no T cell recognizable epitope peptide immobilized thereon or encapsulated therein), the polysaccharide preferably providing a skeleton. The biodegradable polysaccharide nanoparticle of the invention may contain a component other than the saccharide in the skeleton or other portion thereof, or need not have such component.

Biodegradable nanoparticles made chiefly from a poly(organic acid) (hereinafter referred to as "biodegradable poly(organic acid) nanoparticles") may contain a natural poly(organic acid), modified poly(organic acid), synthetic poly(organic acid) or a mixture of such acids (while polypeptide as the main material has been described above). In view of safety and toxicity, more preferable are those comprising a natural poly(organic acid). Examples of preferred biodegradable poly(organic acid) nanoparticles comprising such a natural poly(organic acid) are poly(lactic acid) nanoparticles, etc. Further biodegradable poly(organic acid) nanoparticles may comprise a single organic acid, or at least two organic acids. The biodegradable poly(organic acid) nanoparticle may comprise component poly(organic acids) all linked by the same bond, or the component acids may be linked by different bonds locally or wholly. The poly(organic acids) may be bonded by a linker. The biodegradable poly(organic acid) nanoparticle of the present invention consists mainly of a poly(organic acid) (preferably in an amount of at least 50 wt. % when having no T cell recognizable epitope peptide immobilized thereon or encapsulated therein), the poly(organic acid) preferably providing a skeleton. The biodegradable poly(organic acid nanoparticle of the invention may contain a component other than the poly(organic acid) or amino acid in the skeleton or other portion thereof, or need not have such component.

The biodegradable nanoparticles for use in the present invention are not limited particularly in shape and are generally spherical and usually 80 nm to 100 µm, preferably 100 nm to 50 µm, in size. When so sized, the particles can be given, for example, an increased surface area per unit weight, thereby permitting the T cell recognizable epitope peptide to be immobilized in an increased amount, made retainable in tissues more effectively and to be taken into cells in controllable manner, hence advantageous effects. When otherwise shaped, nanoparticles are sized substantially the same as spherical nanoparticles.

The biodegradable nanoparticles for use in the present invention can be prepared by using known methods. Examples of preparation methods are submerged drying method, spray drying method, spherical crystallization method, solvent replacement method (precipitation/dialysis method), direct ultrasonic dispersion method, etc. For example, biodegradable nanoparticles comprising poly(γ-glutamic acid) and those comprising poly(ε-lysine) can be prepared by the solvent replacement method. Biodegradable polysaccharide nanoparticles can be prepared, for example, by the direct dispersion method. Biodegradable poly(organic acid)nanoparticles can be prepared, for example, emulsion-submerged drying method. Such method are suitably selected and used in combination to provide biodegradable nanoparticles which are adjusted or controlled in material, components, molecular weight, size, electric charge and other parameters in conformity with the purpose. When desired, nanoparticles may be joined by matrix cross-linking.

Various T cell recognizable epitope peptides are usable for immobilization on or encapsulation in biodegradable nanoparticles. Preferable as T cell recognizable epitope peptides are cedar pollen T cell recognizable epitope peptides. These peptides include, for example, P1: 277-290 (KQVTIRIGCKTSSS) (Yoshitomi T et al: Immunology 10)7: 517-520, 2002) which is BALB/c mouse T cell recognizable epitope peptide for Cryj1, P2: 70-83 (HFTFKVDGIIAAYQ) and P2: 246-259 (RAEVSYVHVNGAKF (Yoshitomi T at al: Immunology 107: 517-520,2002, Hirahara S et al; J. Allery Clin. Immunol. 102: 961-967, 1998, Murasugi T et al; Eur. J. Pharmacol. 510: 143-148, 2005) which are BALB/c mouse T cell recognizable epitope peptidea for Cryj2, human T cell recognizable epitope peptides for Cryj1 reported in p16-30, p81-95, p91-105, p106-120, p111-125, p151-165, p156-170, p191-205, p211-225, p231-245, p301-315, p316-330, p331-345, etc, human T cell recognizable epitope peptides for Cryj2 reported in p66-80.p76-90, p81-95, p96-107, p141-155, p146-160, p181-195, p186-200, p236-250, p336-350, p346-360, p351-365 (Sone T et al; J. Immunol. 161:448-457, 1998, Hirahara K et al: J. Allergy Clin. Immunol. 108: 94-100. 2001), etc. More preferable are human or mouse cedar T cell recognizable epitope peptide. These peptides are used singly or in combination as T cell recognizable epitope peptides of the invention.

A suitable T cell recognizable epitope peptide can be selected for immobilization on or encapsulation in biodegradable nanoparticles, in accordance with the state of the subject of administration, for example, the kind, age, body weight and health condition of the animal, the kind of disease to be prevented, and/or already developed and to be treated, or the cause thereof. Biodegradable nanoparticles may have immobilized thereon or encapsulated therein a single kind of or at least two kinds of T cell recognizable epitope peptides.

The T cell biodegradable epitope peptide can be immobilized on or encapsulated in biodegradable nanoparticles by various known methods. The epitope peptide may be immobilized on or encapsulated in biodegradable nanoparticles directly or by means of a linker such as polyethylene glycol (PEG). Peptides are immobilized or encapsulated by known methods, such as the bonding method using covalent bonds, ionic bonds or intermolecular forces, adsorption method or inclusion method. For example, the functional group on the biodegradable nanoparticle may be linked to the functional group of the peptide by a covalent bond for immobilization or encapsulation. The immobilization or encapsulation may be effected by an ionic bond when the charge on the nanoparticle is opposite to that of the peptide. For example, the peptide can be immobilized on the biodegradable poly(γ-glutamic acid) nanoparticle by the inclusion method by introducing a hydrophobic amino acid into poly(γ-glutamic acid) by covalent bonds, dissolving the resulting acid in an organic solvent and subsequently adding an aqueous solution of the peptide dropwise to the solution. Alternatively, the peptide may be immobilized on or encapsulate in biodegradable nanoparticles by a suitable combination of the bonding method, adsorption method and/or inclusion method. Such a mode of immobilization or encapsulation can be suitably selected in accordance with the purpose of use (e.g., the kind of subject or disease).

The biodegradable nanoparticles of the invention having a T cell recognizable epitope peptide immobilized thereon or encapsulated therein have the advantage that the peptide remains unaffected in its stereostructure, such that the immobilized or encapsulated peptide is less likely to alter in amount or properties even after freeze-drying and can be preserved for a prolonged period of time.

In another aspect of the present invention, the invention provides an immunotherapeutic agent comprising a biodegradable nanoparticle having the peptide immobilized thereon or encapsulated therein. The biodegradable nanoparticles having the immobilized or encapsulated T cell recognizable epitope peptide are usable as an immunotherapeutic agent.

It is the biodegradable nanoparticle that is used in the immunotherapeutic agent of the invention as a carrier or adjuvant for immobilizing or encapsulating the T cell recognizable epitope peptide. The nanoparticle is eventually decomposed in the living body with a decomposition enzyme. The immunotherapeutic agent of the present invention comprises a biodegradable nanoparticles having a T cell recognizable epitope peptide immobilized thereon or encapsulated therein, an excipient or carrier, and when desired, other components such as a suspending agent, isotonic agent and antiseptic. Examples of excipients or carriers are aqueous media such as water, ethanol and glycerin, and nonaqueous media such as fatty acids, fatty acid esters and like oils or fats. The immunotherapeutic agent of the invention may be in a form of preparation, as selected according with factors such as the state of the subject and the kind of disease. The agent is, for example, a suspension in an aqueous carrier, or in the form of a powder, capsules or tablets. The immunotherapeutic agent prepared by freeze-drying may be used as suspended in a suitable excipient or carrier before administration. The method and route of administration of the immunotherapeutic agent of the invention are not limited particularly but can be selected according to factors such as the form of preparation, state of the subject and kind of the disease. The agent of the invention may be given to the subject for example, by injection, parenterally or orally.

Furthermore, the rate and duration of release of the T cell recognizable epitope peptide are controllable by changing the material or component of the biodegradable nanoparticle and varying the molecular weight, size and other parameters thereof. The method to be practiced for this purpose is also known in the art. In the case of nanoparticles comprising a graft copolymer of poly(γ-glutamic acid) and hydrophobic amino acid, delayed release immunotherapeutic agent is available, for example, by controlling the kind and content of the hydrophobic amino acid. Furthermore, a bond decomposable with an enzyme locally present in a specific organ or part may be introduced into the peptide-nanoparticle bond or into the nanoparticle so as to render the peptide releasable in the specific organ or part.

The immunotherapeutic agent of the present invention can be administered to various subjects in order to prevent and/or treat various diseases. The subjects to be given the immunotherapeutic agent of the invention are not limited specifically but are preferably mammals, more preferably humans.

Although the disease to be prevented and/or treated with the immunotherapeutic agent of the invention is not limited particularly, preferable are pollinosis, year-round nasal allergic disease, seasonal nasal allergic disease, etc., among which pollinosis is desirable.

### BRIEF DESCRIPTION OF THE DRAWINGS

FIG. 1 is a graph showing the immunity inducing effect of biodegradable nanoparticles.
FIG. 2 is a graph showing the immunity inducing effect of Th1 cytokine due to hypodermic administration.
FIG. 3 is a graph showing the immunity regulating effect due to ophthalmic administration.
FIG. 4 is a graph showing the therapeutic effect due to nasal administration.

### BEST MODE OF CARRYING OUT THE INVENTION

The present invention will be described below in greater detail with reference to Test Examples and Examples. The invention, however, is not limited to these examples.

### Example 1

(1) Preparation of mouse cedar pollen T cell recognizable epitope peptides as converted to PEG (polyethylene glycol)
   P1: 277-290; KQVTIRIGCKTSSS (hereinafter referred to as "P1") was selected as BALB/c mouse T cell recognizable epitope peptide for Cryj1, and P2: 246-259; RAEVSYVHVNGAKF (hereinafter referred to as "P2") was selected as a BALB/c mouse T cell recognizable epitope peptide for Cryj2. The conversion of these peptides to PEG (polyethylene glycol) was made by Toray Research Center Co. Ltd. Mouse cedar pollen T cell recognizable epitope peptides as converted to PEG (polyethylene glycol) were prepared by the Fmoc solid-phase process.
(2)Preparation of biodegradable nanoparticles having mouse cedar pollen T cell recognizable epitope peptide immobilized thereon
   Poly(γ-glutamic acid) (γ-PGA, 300,000 in molecular weight) in an amount of 607 mg (4.7 unit mmols) was dissolved in 100 ml of 54 mM aqueous solution of sodium hydrogencarbonate (pH 8.5). Subsequently added to the solution were 901 mg (4.7 mmols) of 1-ethyl-3-(3-dimethylaminopropyl)carbodiimide hydrochlorate (WSC) and 1080 mg (4.7 mmols) of L-phenylalanine ethyl ester (L-PAE), and the mixture was reacted overnight at room temperature with stirring. The resulting solution was dialyzed for 3 days using a dialysis membrane (molecular weight fraction 50,000), followed by lyophilization. To the lyophilized dry product was added 100 ml of ethanol, and the mixture was stirred overnight. The resulting solution was centrifuged (1,500× g, for 20 minutes), and the precipitate was dried in a vacuum, giving a graft copolymer of poly(γ-glutamic acid) and phenylalanine ethyl ester (γ-PGA-g-L-PAE). The γ-PGA-g-L-PAE was dissolved in DMSO to a concentration of 10 mg/ml, and the solution was added dropwise to a saline in an amount equal to that of the solution to obtain nanoparticles, followed by dialysis and lyophilization for the preparation of the particles. To a dispersion of 20 mg/ml of γ-PGA nanoparticles was added an aqueous solution of 1 mg/ml of WSC (20 mM phosphoric acid buffer, pH 5.8) in an amount equal to tat of the dispersion, followed by a reaction at room temperature for 20 minutes. After the reaction of specified period of time, the reaction mixture was centrifuged at 14,000 × g for 15 minutes to remove the WSC solution, and a solution of 1 mg/ml of peptide as dissolved in PBS was added to the product so that the concentration of nanoparticles would be 5 mg/ml, followed by a reaction overnight at 4° C. The peptide solution was removed from the resulting reaction mixture by centrifuging, and the solid product was dispersed in water again. This procedure was repeated to remove the untreated peptide and to obtain a dispersion of 10 mg/ml of nanoparticles having the peptide immobilized thereon. The amount of peptide supported on the nanoparticles was quantitatively determined by the Lowry method.

### Example 2

### Immunization potential of biodegradable nanoparticles having immobilized thereon mouse cedar pollen T cell recognizable epitope peptide

Subcutaneously injected into the footpads of BALB/c mice (6-week-old male) was 100 µl (50µl for each footpad) of a suspension of biodegradable nanoparticles having immobilized thereon Cryj1 or Cryj2 T cell recognizable epitope peptide (P1 or P2) (the amount of nanoparticles corresponding to 20 µg of the peptide) or nanoparticles having no peptide immobilized thereon. On day 11, a draining lymph node was removed to collect lymph node cells, which were then suspended in a mixture of RPM 11640 medium and 10% fetal calf serum (FCS). Such cells from two mice were combined together for use in each group. The cells were placed onto a 96-well incubation plate in an amount of 5 ×10⁵ in each well. Further placed into each well as an antigen stimulus was P1 or P2 to a final concentration of 20 µg/ml or a cedar pollen roughly purified antigen (Sugi Basic Protein, SBP, Asahi Food and Health Care Co., Ltd.) in an amount of 10 µg/ml. Half of the mixture to be incubated was replaced by a fresh mixture to be incubated and containing [³H] tritium-thymidine (0.5 µCi) 48 hours after the start of incubation. The cells were then collected onto a glass filter 16 hours thereafter by a cell harvester, and the [³H] tritium intake (thymidine intake) was measured by a liquid scintillation counter for the evaluation of cell proliferation. The SBP mentioned above is an allergen containing both Cryj1 and Cryj2.

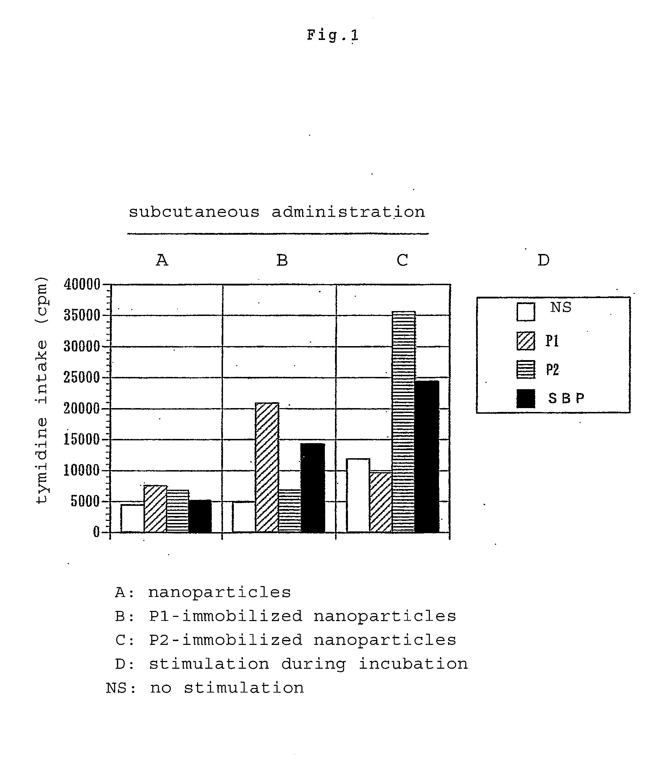

FIG. 1 shows the result. A specific cell proliferation response was observed to each of T cell recognizable epitope peptides (P1 and P2) injected first. Also observed was a response to the SBP containing both of the epitope peptides. No response was found to the nanoparticles having no peptide immobilized thereon.

These results indicate that T cell recognizable epitope peptide as immobilized on nanoparticles is capable of inducting specific immune response to antigens, consequently revealing that the T cell recognizable epitope peptide as immobilized on nanoparticles remains free of degradation or decomposition.

### Example 3

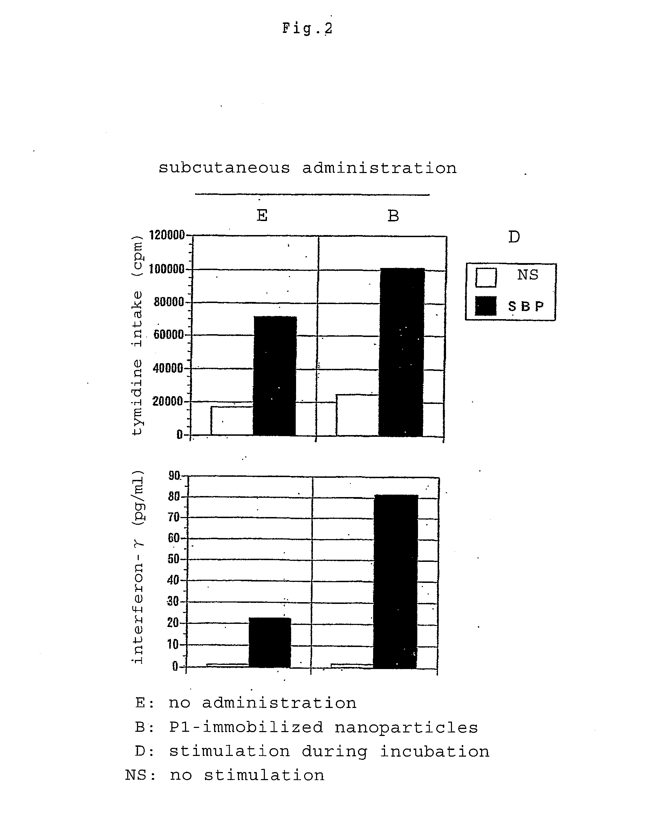

Immunomodulating effect of biodegradable nanoparticles having immobilized thereon mouse cedar pollen T cell recognizable epitope peptide
(1) Th1 cytokine immunity induction by subcutaneous administration
   Subcutaneously injected into the footpads of BALB/c mice (10-week-old male) was 100 µl (50 µl for each sole) of a suspension of biodegradable nanoparticles having immobilized thereon Cryj1 T cell recognizable epitope peptide (P1) (the amount of nanoparticles corresponding to 50 µg of the peptide). On day 16, a suspension of 5 µg of cedar pollen roughly purified antigen SBP in Freund's incomplete adjuvant was injected into the right footpads to give rise to an immune response. Five days thereafter, draining lymph node cells were collected, placed onto a 96-well incubation plate in the same manner as in Example 2 and stimulated with cedar pollen roughly purified antigen SBP at a final concentration of 10 µg/ml. The proliferation of cells was measured by the same method as described in Example 2. For the measurement of Th1 cytokine production, the supernatant was collected 64 hours after the start of incubation to quantitatively determine interferon-γ by sandwich ELISA using Bio-Plex cytokine assay system (product of Bio-Rad).
   The result is shown in FIG. 2. With respect to cell proliferation response (thymidine intake) to the stimulation with the antigen, the cells from the mice subcutaneously given nanoparticles having P1 immobilized thereon were only slightly greater than the cells from the mice given no subcutaneous injection, and were not greatly different from the latter.
   On the other hand, remarkably increased production of interferon-γ was found in the cells from the mice subcutaneously given nanoparticles having P1 immobilized thereon.
   These results indicate the nanoparticles having the T cell recognizable epitope peptide remarkably increase the production of interferon-γ.
(2) Immunomodulation by mucosal administration
   Mucosal administration of antigens is thought promising in immunotherapy, and the conjunctiva is considered to be one of the routes of administration (J. Immunol. 2000, 164: 4543-4550). Biodegradable nanoparticles having immobilized thereon T cell recognizable epitope peptide were ocularly-instilled to investigate the influence on the response to the subsequent antigen sensitization.
   A saline solution of Cryj2 T cell recognizable epitope peptide (P2) (5 µg of the peptide) or a suspension of P2 immobilized nanoparticles (corresponding to 5 µg of the peptide) administered dropwise, in an amount of 10 µl, to the conjunctivas of the eyes of BALB/c mice (7-week-old male) eight times (on days 1-4 and days 6-9). On day 16, a suspension of 5 µg of cedar pollen roughly purified antigen SBP in Freund's incomplete adjuvant was injected for antigen sensitization into the right footpads to give rise to an immune response. Five days thereafter, draining lymph node cells were collected, placed onto a 96-well incubation plate in the same manner as in Example 2 and stimulated with cedar pollen roughly purified antigen SBP at a final concentration of 10 µg/ml. The proliferation of cells was measured by the same method as described in Example 2. For the measurement of cytokine production, the supernatant was collected 64 hours after the start of incubation to quantitatively determine intetrleukin-5 and interferon-γ by sandwich ELISA using Bio-Plex cytokine assay system (product of Bio-Rad).

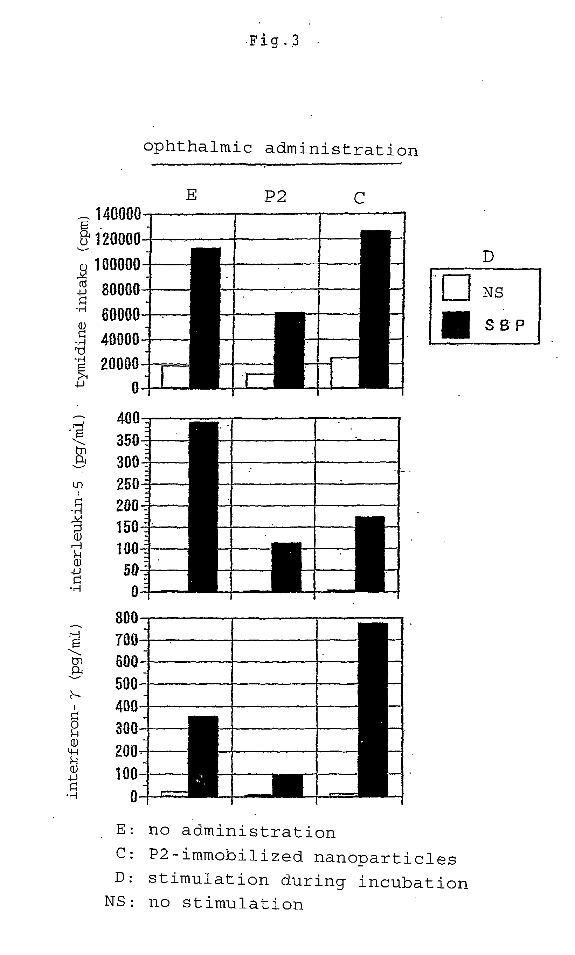

The result is shown in FIG. 3. As compared with the cells from the ocularly untreated mice with respect to cell proliferation response (thymidine intake) to the stimulation with the antigen, the cells from the mice ocularly given P2 alone were lower, while the cells ocularly given P2 immobilized nanoparticles remained almost unaltered.

With respect to the production of interleukin-5 which is Th2 cytokine, the cells from the mice ocularly given P2 alone and the cells ocularly given P2 immobilized nanoparticles were found reduced to nearly the same extent from the level of the cells from the ocularly untreated mice.

As compared with the cells from the ocularly untreated mice with respect to the production of interferon - γ which is Th1 cytokine, the cells from the mice ocularly given P2 alone were lower but the cells ocularly given P2 immobilized nanoparticles were found increased.

These results indicate that the single administration of the T cell recognizable epitope peptide reduces the production of both Th2 cycokine and Th1 cytokine, and that the administration of nanoparticles having the T cell recognizable epitope peptide immobilized thereon diminishes the production of Th2 cytokine while increasing the production of Th1 cytokine. Thus, it is suggested that nanoparticles having the T cell recognizable epitope peptide immobilized thereon controls the balance between Th1 and Th2 and is useful as an immunotherapeutic agent.
(1) Therapeutic effect by nasal administration
   Investigations were made into the activity of nanoparticles having the T cell recognizable epitope peptide immobilized thereon to be exerted on inflammatory cell infiltration in the case where an antigen is intratracheally given to mice sensitized to cedar pollen.

BALB/c mice (9-week-old male, six in each group) were intraperitoneally given a suspension of 5 µg of cedar pollen roughly purified antigen SBP in 2 mg of alum twice at an interval of 1 week for immunization. Two weeks after the final immunization, suspensions of nanoparticles (P1-NP)having Cryj1 T cell recognizable epitope peptide (P1) immobilized thereon were nasally given (to both nares) under anesthesia in an amount of 20 µl 3 times every other day, the suspensions corresponding to 40 µg and 4 µg, respectively.
A phosphate buffer (PBS) serving as a vehicle was given to an untreated group (Sham group). Five weeks after the final nasal administration, 2µg of cedar pollen roughly purified antigen Cryj1 was intratracheally given to the animals under anesthesia four times (once daily, for 3 consecutive days, and further once 3 days later). In place of the antigen, PBS was administered to a negative control group (Sham/PBS group). The mice were anesthetized to death two days after the intratracheal administration, the trachea was exposed and cut open, a broncheal cannula was installed, and alveoli were washed three times with 1 ml of PBS containing 0.1% of bovine serum albumin (BSA) to obtain bronchoalveolar lavage fluid (BALF). The BALF obtained was centrifuged, the solids were suspended in PBS containing 0.1% of BSA, the total number of leukocytes was counted by a cytometer and cell smear specimens were prepared. The specimens were stained with a Wright-Gimusa stain, and the cells were divided into eosinophils, neutrophils and mononuclear leukocytes including lymphocytes, monocytes and macrophages accoroding to common classification, and at least 300 cells were counted up per specimen to obtain the proportions of the different cells. Infiltrating cell count (×10⁴ cells/BALF) in the collected BALF was calculated from the cell proportions.

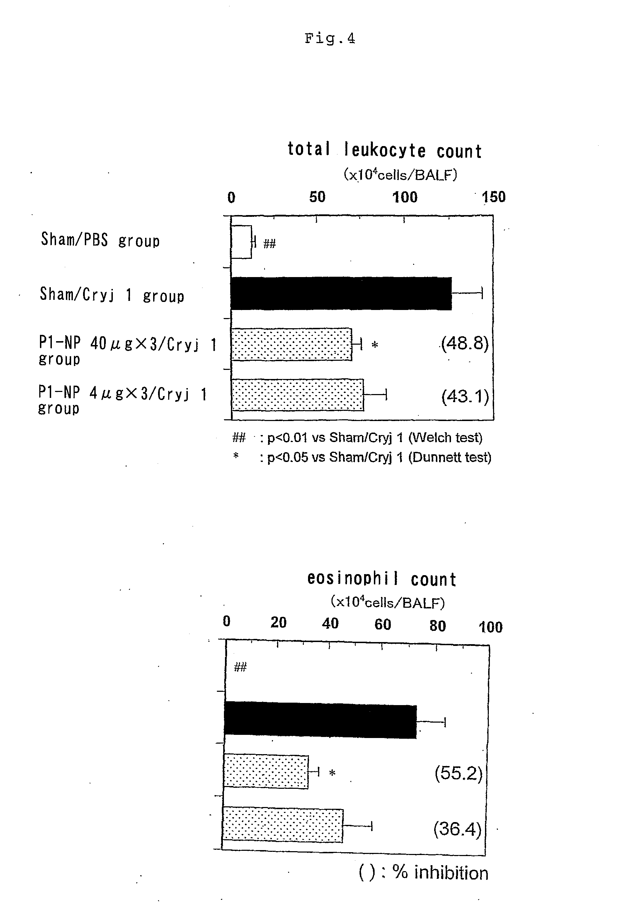

FIG. 4 shows the result. The Sham/Cryj1 group wherein the SBP-immunized mice were intratracheally given Cryj1 was increased in the total leukocyte count in BALF as compared with the Sham/PBS group, and about 60% of the infiltrating cells were found to be eosinophils. On the other hand, the increase in the total leukocyte count in BALF, especially in eosinophil count, was found suppressed dose-dependently in P1-NP groups wherein the mice were nasally given the suspension of P1-immobilized nanoparticles in advance, i.e., in the group given P1-immobilized nanoparticles (P1-NP) at doses of 40 µg and also in the group with doses of 4 µg. Especially, the group with 40-µg doses exhibited a significantly suppressed increase.

These results reveal that nanoparticles having T cell recognizable epitope peptide immobilized thereon exhibits a therapeutic effect even when administered after sensitization has been established against cedar pollen, thus substantiating the usefulness of the immunotherapeutic agent against pollinosis or the like.

### INDUSTRIAL APPLICABILITY

The present invention has made it possible to provide biodegradable nanoparticles having a T cell recognizable epitope peptide, more particularly a T cell recognizable epitope peptide of pollinosis patients, immobilized thereon or encapsulated therein without degradation or decomposition, and/or an immunotherapeutic agent comprising the nanoparticle. Unlike the peptide as administered singly, the peptide as immobilized on or encapsulated in biodegradable nanoparticles controls the Th1/Th2 balance.

The biodegradable nanoparticles of the invention are therefore useful as an immunotherapeutic agent for treating, for example, pollinosis, year-round nasal allergic disease
and seasonal nasal allergic disease.

## Claims

1. A biodegradable nanoparticle having a T cell recognizable epitope peptide immobilized thereon or encapsulated therein.

2. A nanoparticle according to claim 1 which comprises a nanoparticle mainly prepared from a polypeptide, polysaccharide or poly(organic acid).

3. A nanoparticle according to claim 2 wherein the polypeptide is poly(γ-glutamic acid).

4. A nanoparticle according to claim 2 wherein the polypeptide is a graft copolymer of poly(γ-glutamic acid) and phenylalanine ethyl ester.

5. A nanoparticle according to any one of claims 1 to 4 wherein the T cell recognizable epitope peptide is encapsulated in the nanoparticle.

6. A nanoparticle according to any one of claims 1 to 4 wherein the T cell recognizable epitope peptide is present on a surface of the nanoparticle.

7. A nanoparticle according to any one of claims 1 to 6 wherein the T cell recognizable epitope peptide is a cedar pollen T cell recognizable epitope peptide.

8. An immunotherapeutic agent containing a nanoparticle according to any one of claims 1 to 7.

9. An immunotherapeutic agent according to 8 for treating and/or preventing cedar pollinosis.

10. Use of a biodegradable nanoparticle having a T cell recognizable epitope peptide immobilized thereon or encapsulated therein for preparing an immunotherapeutic agent.

11. Use according to claim 10 for treating and/or preventing cedar pollinosis.

12. An immunotherapy comprising administering to a mammal an effective amount of a biodegradable nanoparticle having a T cell recognizable epitope peptide immobilized thereon or encapsulated therein.

13. An immunotherapy according to claim 12 for treating and/or preventing cedar pollinosis.

## Amended claims

### Amended claims under Art. 19.1 PCT

**1.** (after amendment)
A biodegradable nanoparticle having a cedar pollen T cell recognizable epitope peptide immobilized thereon or encapsulated therein.

**2.** (after amendment)
A nanoparticle according to claim 1 which comprises a nanoparticle mainly prepared from a poly(γ-glutamic acid).

**3.** (deleted)

**4.** (after amendment)
A nanoparticle according to claim 1 which is a graft copolymer of poly(γ-glutamic acid) and phenylalanine ethyl ester.

**5.** (deleted)

**6.** (deleted)

**7.** (deleted)

**8.** (after amendment)
An immunotherapeutic agent containing a nanoparticle according to any one of claims 1, 2 and 4.

**9.** An immunotherapeutic agent according to 8 for treating and/or preventing cedar pollinosis.

**10.** (after amendment)
Use of a biodegradable nanoparticle having a cedar pollen T cell recognizable epitope peptide immobilized thereon or encapsulated therein for preparing an immunotherapeutic agent for treating and/or preventing cedar pollinosis.

**11.** deleted)

**12.** An immunotherapy comprising administering to a mammal an effective amount of a biodegradable nanoparticle having a T cell recognizable epitope peptide immobilized thereon or encapsulated therein.

**13.** An immunotherapy according to claim 12 for treating and/or preventing cedar pollinosis.

**14.** (added)
Use of a nanoparticle for preparing an immunotherapeutic agent according to claim 10 which uses a nanoparticle mainly prepared from a poly(γ-glutamic acid).

**15.** (added)
Use of a nanoparticle for preparing an immunotherapeutic agent according to claim 10 which uses a nanoparticle which is a graft copolymer of poly(γ-glutamic acid) and phenylalanine ethyl ester.
